# EUROPÄISCHE PATENTSCHRIFT

(11) **EP 0 928 212 B1**
(45) Veröffentlichungstag und Bekanntmachung des Hinweises auf die Patenterteilung: **02.10.2002**
(21) Anmeldenummer: 95925870.8
(22) Anmeldetag: 13.07.1995
(51) Int. Cl.: A61N 1/05

(54) **FLEXIBLE KÜNSTLICHE NERVENPLATTE**
FLEXIBLE ARTIFICIAL NERVE PLATE
PLAQUETTE ARTIFICIELLE SOUPLE DE REMPLACEMENT DE NERFS

(30) Priorität: 13.07.1994 DE 4424697
(43) Veröffentlichungstag der Anmeldung: 14.07.1999
(73) Patentinhaber: Fraunhofer-Gesellschaft zur Förderung der angewandten Forschung e.V., 80636 München (DE)
(72) Erfinder: MEYER, Jörg-Uwe, D-66386 St. Ingbert (DE); STIEGLITZ, Thomas, D-66954 Pirmasens (DE)
(86) Internationale Anmeldenummer: EP9502754
(87) Internationale Veröffentlichungsnummer: WO96002298

(56) Entgegenhaltungen:
- WO-A-93/20887
- US-A- 3 738 368
- US-A- 3 955 560
- IEEE TRANSACTIONS ON BIOMEDICAL ENGINEERING, Nr. 9, September 1992 NEW YORK USA, Seiten 893-902, XP 000322966 G. T. A. KOVACS, C.W. STORMENT, J. M. ROSEN 'Regeneration Microelectrode Array for Peripheral Nerve Recording and Stimulation'
- PROCEEDINGS OF THE ANNUAL INTERNATIONAL CONFERENCE OF THE IEEE ENGINEERING IN MEDICINE AND BIOLOGY SOCIETY, Bd. 15, Nr. 1, 28. - 31.Oktober 1993 SAN DIEGO,CALIFORNIA USA, Seiten 1247-1248, XP 000452850 D.J.TYLER, D. DURAND 'Design and acute test of a radially penetrating interfascicular nerve electrode'

## Beschreibung

Die Erfindung betrifft eine flexible und nicht leitende, künstliche implantierbare Nervenplatte (FNP) zum Einlegen und Einfügen zwischen die Faszikel eines Nervenbündels.

### Technisches Anwendungsgebiet

Das technische Anwendungsgebiet der Erfindung ist in der Neuroprothetik zu finden. Hier werden neue Möglichkeiten gesucht, Nervenbündel und -fasern möglichst schonend mit einer Vielzahl von Elektroden zu kontaktieren, um multilokal und simultan Nervensignale abzuleiten und/oder Nervenfasern zu stimulieren. Dadurch sollte es möglich sein, durch den Einsatz von technischen Hilfssystemen ausgefallene Körperfunktionen durch Registrierung von Nervensignalen und durch Stimulation von Nerven im peripheren Nervensystem wieder herzustellen.

### Stand der Technik; Nachteile des Standes der Technik

### Intrafaszikuläre Elektroden

Zur Erfassung von Nervensignalen innerhalb eines Faszikels (Faszikel ist eine organisatorische Einheit aus Nervenfasern innerhalb eines Nervenbündels) werden intrafaszikuläre Ableittechniken verwendet. Bei den intrafaszikulären Elektroden handelt es sich um bipolare Einzelelektroden, die sowohl das elektrische Potential im Nervenfaszikel als auch das Potential an der Nervenoberfläche ableiten. Hierdurch wird eine differentielle Messung möglich, die die durch elektromagnetische Einstreuungen hervorgerufenen Störungen weitgehend eliminiert. Mit implantierten intrafaszikulären Elektroden wurden die Signale sensorischer Tastnerven von Katzen-Vorderpfoten aufgezeichnet. Weiterhin wurden mittels implantierter intrafaszikulärer Elektroden erfolgreich motorische Nerven stimuliert.

Nachteile: Der operative und zeitliche Aufwand zur genauen Plazierung der Elektroden ist beträchtlich. Die Elektroden sind als Drahtelektroden einkanalig. Neue mehrkanalige Ausführungsformen in Silizium sind in der Entwicklung. Einund mehrkanalige Ausführungsformen sind für die dauerhafte Implantation nur ungenügend geeignet. Zudem wird bei Einbringen der Elektroden das Perineurium (Membran um die Faszikel) verletzt.

Aus IEEE Transactions on Biomedical Engineering vol 39, Nr.3, 1992, S.271-279 und Nr.5, 1992, S.474-481 sind Schaftelektroden bekannt, die speziell für die Bedürfnisse einer intracortikalen, d.h. die harte Hirnhaut der Großhirnrinde durchdringenden Implantation bzw. einer transmuralen, intrakardialen Implantation entwickelt worden sind. Ein Einsatz einer 125 µm dicken Kapton-Folie in peripheren Nerven zwischen den Faszikeln ist undenkbar, da aufgrund der Bewegung der Elektrode durch die Muskelbewegung sowie durch die Gewichtsund Momentenbelastung des Nervsvon der Mikroelektrode aufgrund der Schwerkraft der Nerv dermaßen stark degenerieren wird, daß eine Ableitung und Simulation nicht mehr möglich ist.

### Maschenelektroden

Einzelfaser-Kontaktierung von aussprossenden proximalen Nervenstümpfen in maschenähnliche Mikrostrukturen konnte bereits in der oben angeführten Literaturstelle nachgewiesen werden. Um die Sprossung des Nervenstumpfes zu begünstigen, ist es notwendig, eine Resektion (Entfernung des fibrös veränderten Nervenabschnittes durch Schneiden mit Skalpell) am proximalen Stumpf vorzunehmen. Dieser Eingriff scheint bei Kontaktierung von größeren peripheren Nerven (z.B. n. ischiaticus, n. femoralis) oder von Nerven ohne totalen Kontinuitätsverlust nicht geeignet. Maschenelektroden sind für die Kontaktierung von gesunden Nerven nicht geeignet, da zur Durchsprossung freie Nervenendigungen vorhanden sein müssen.

### Manschettenelektroden

Seit Ende der 70^{er} Jahres wird versucht, mit extraneuralen manschettenartigen Elektroden, sogenannten Cuff-Elektroden, die zirkulär um den Nerv gelegt werden, Nervenbündel zu stimulieren und Neurogramme aufzunehmen. Die Manschettenelektroden sind inzwischen zu den erfolgreichsten Bauformen biomedizinischer Elektroden geworden und werden in vielen Bereichen eingesetzt. Es bestehen einige US-Patente für verschiedene Bauformen: 3,774,618, 3,738,368, 3,654,933. Einige Elektroden sind seit 15 Jahren sicher im Einsatz. Die Anzahl der Elektroden beschränkt sich auf maximal 3 - 4. Es kann nur eine begrenzte örtliche Auflösung der Stimulation innerhalb des Nervens erreicht werden (tripolare Elektroden). Nachteil: Eine multilokale Stimulation (mit mehr als 4 Elektroden) des Nervens ist mit diesen Elektroden nur begrenzt möglich. Tiefere Strukturen können nur mit erhöhtem Reizstrom erreicht werden. Eine gleichzeitige Stimulation und Ableitung ist nicht möglich.

Aufgabe der Erfindung ist es daher, mit einem Implantat die gleichzeitige Stimulation der Nerven und Ableitung der Nervensignale zu ermöglichen. Diese Aufgabe wird erfindungsgemäß durch die Nervenplatte nach Anspruch 1 sowie ein Verfahren zur Herstellung einer Nervenplatte nach Anspruch 10 gelöst, vorteilhafte Ausgestaltungen sind in den Unteransprüchen gekennzeichnet.

Mittels der Erfindung und durch eine beidseitige Elektrodenanordnung und eine interfaszikuläre Implantation gelingt eine direkte Multikontaktierung von Faszikeln. Um diesen Vorteil offenzulegen, muß ein kurzer Exkurs in die Neurophysiologie unternommen werden. Ein Faszikel ist ein Faserbündel, das vom sogenannten Perineurium, einer Art umhüllenden Schicht, umgeben ist, die im Vergleich zum Epineurium, welches die Faszikel -bildlich gesprochen- zu einem Nerv zusammenfaßt, härter ist und auch eine Schutzschicht für die Nervenfasern darstellt. Das Implantationskonzept der Nervenplatte sieht eine Implantation zwischen den Faszikeln vor, bei der das Perineurium unbeschädigt bleibt und daher eine geringere Traumatisierung des Nervs bzw. der Nervenfasern zu erwarten ist als bei Elektrodenkonzepten, bei denen Schaftelektroden in den Nerven bzw. in den Faszikel gestochen werden, wodurch eine Verletzung des Perineuriums prinzipiell in Kauf genommen wird. In distalen Teilen peripherer Nerven sind diese Faszikel funktionell geordnet d.h. bestimmte Nervenfaserbündel sind bestimmten Organen bzw. Körperteilen zugeordnet. Proximal-peripher und zentral ist diese Trennung oft nicht eindeutig. Durch die Konzeption der Nervenplatte können so über die Faszikel gezielt funktionelle Körperteile bzw. Organe angesprochen werden. Durch die Implantation einer flachen, flexiblen Struktur im Nerven -und nicht wie bei Manschettenelektroden um den Nerven herum- können auch tiefe Strukturen mit geringen Strömen stimuliert werden, so daß eine funktionelle Schädigung des Nervs durch hohe Ströme aufgrund des Konstruktionskonzeptes ausgeschlossen werden kann. Ferner können Druckschäden durch postoperative Ödeme am Nerven, wie sie bei Manschettenelektroden auftreten können, durch dieses Konstruktionsprinzip am peripheren Nerven vermieden werden.

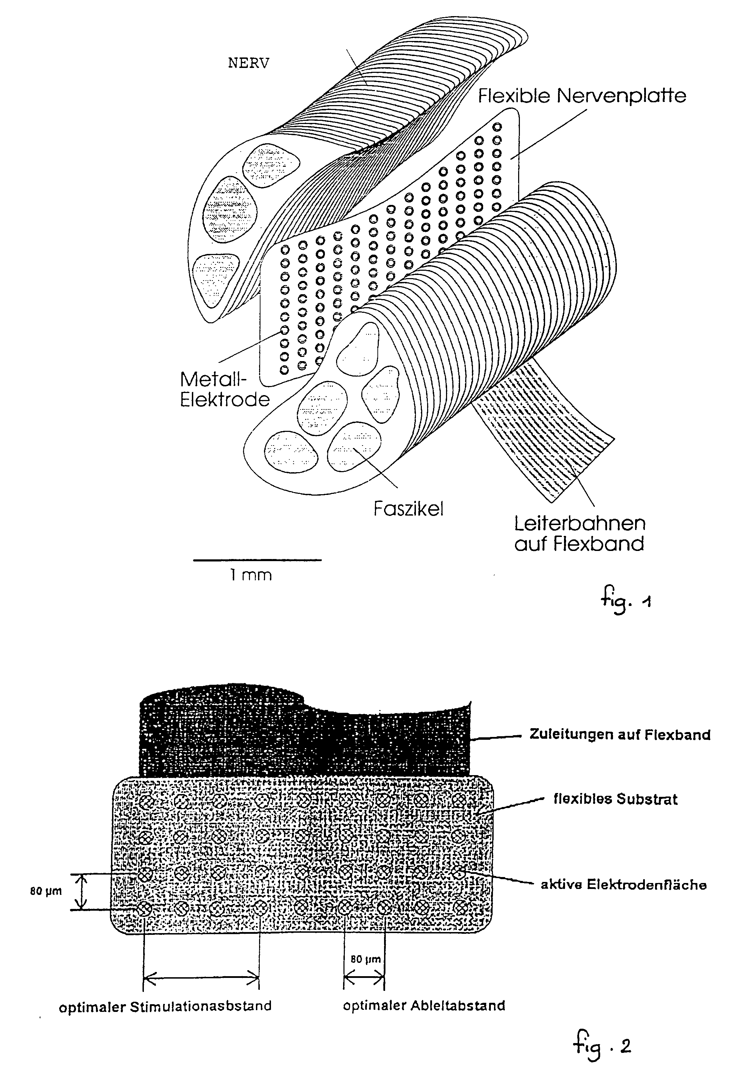

Durch die beidseitige Anordnung von Elektroden ist eine gleichzeitige Stimulation (auf der einen Plattenseite) und Ableitung von Nervensignalen (auf der anderen Plattenseite) möglich. Werden simultan von mehreren Elektroden Elektroneurogramme abgeleitet, kann das Signal zeitlich-räumlich aufgelöst werden. Mittels der vielen Elektroden auf beiden Seiten kann auch eine Stimulation und Ableitung der Nervensignale gleichzeitig auf beiden Seiten bei entsprechendem Abstand der Elektroden bzw. Elektrodenblöcke erfolgen, siehe hierzu die Abbildung unten. Hierdurch wird die Möglichkeit verbessert, viele Nervenenden zu erfassen bzw. zu kontaktieren.

Bisherige Elektrodenansätze, wie oben zum Stand der Technik zitiert, verwenden stets nur einseitig Elektroden, da in klassischer Siliziumtechnologie das Dünnen der Wafer durch einen selektiven, naßchemischen Ätzprozeß vorgenommen wird, bei dem die Elektrodenstruktur zu Prozeßbeginn durch eine Eindiffusion von Bor in das Siliziumsubstrat definiert werden muß. Es ist nicht möglich, nach diesem Diffusionsschritt in der Substrattiefe an der unteren Diffusionsfront Elektroden auf- bzw. einzubringen. Nach dem Freiätzen der Proben mittels EDP ist es ebenfalls nicht mehr möglich, mit den Methoden der Halbleitertechnologie und Mikromechanik, in Dünnfilmtechnik Metall aufzubringen und zu strukturieren, es mit einer isolierenden Deckschicht zu versehen und danach Elektrodenfenster zu öffnen. Die Abkehr von Silizium zu polymeren Werkstoffen (Polyimid) und die Entwicklung eines Prozesses, beidseitig Elektroden auf einer flachen Struktur in Multilayertechnik zu erstellen, stellt daher eine große Verbesserung gegenüber dem Stand der Technik dar.

### Mit der Erfindung gelöste Aufgaben

Mit der implantierbaren, flexiblen Nervenplatte ist es möglich, entlang mehrerer Faszikel oder Nervenfasern dauerhaft multilokal und simulatan Nervensignale abzuleiten und mit geringer Reizintensität Nerven zu stimulieren ohne den Nerven zu durchtrennen oder nachhaltig zu traumatisieren. Dies ist Voraussetzung für eine multifunktionale Neuroprothese.

Die FNP mißt Summenaktionsimpulse im Interfaszikulärraum und kann auch nur dort stimulieren. Ein Einzelfaserkontakt ist nicht errreichbar und nicht beabsichtigt. Der Vorteil der Erfindung ist in der geringen Traumatisierung bei der Implantation zu sehen.

Es kann bei der Elektrodenkonfiguration und dem Entwurf des Reizprotokolls auf Techniken, die ursprünglich für Cuff-Elektroden entwickelt worden sind, zurückgegriffen werden. Der Einsatz von tripolaren Elektroden mit Steuerströmen ermöglicht eine Feldbegrenzung auf die unmittelbare Umgebung der Elektroden, so daß kleine Faserverbändegereizt werden können. Durch differenzierte Reizprotokolle mit unterschwelligen Prä-Pulsen werden sich auch von der Nervenplatte entfernt liegende Faszikel selektiv reizen lassen. Mit dem Einsatz von Blocktechniken (z.B. anodischer Block) bzw. bei gleichzeitiger Stimulation durch mehrere beieinander liegende Elektroden können die Nervenfasern ihre Größe entsprechend errregt werden.

### Erzeugte Verbesserung und Vorteile gegenüber dem Stand der Technik

- Direkte Multikontaktierung von Faszikeln und Nervenfasern innerhalb eines Nervenbündels.
- Gleichzeitige Stimulation (z.B. auf einer Plattenseite) und Ableitung (auf der anderen Plattenseite) möglich.
- Multielektroden befinden sich auf einem flachen, flexiblen Substrat --> geringe Traumatisierung des Nervens.
- Mechanische Stabilität durch Einbettung der Flexiblen Nervenplatte innerhalb des Nervenstrangs.
- Hohe zeit-räumliche Signalauflösung.

Das Konzept der Flexiblen Nervenplatte stellt auch einen neuen Ansatz bezüglich der Technologie der Elektrodenherstellung dar. Die bisher nach dem Stand der Technik in Silizium-Technologie erstellten Mikroelektroden weisen eine klare Trennung von Substrat (mechanische Stabilität), Metallisierung (Elektroden und Zuleitungen) und Isolations- und Passivierungsschichten (Biokompatibilität, Isolation von Leiterbahnen) auf. Durch die Wahl eines flexiblen, isolierenden Materials wie z.B. Polyimid dienen die erstellten Schichten ober- und unterhalb die Elektrodenmetallisierungen gleichzeitig der Isolation und der Herstellung der mechanischen Stabilität.

Die Flexible Nervenplatte besteht aus einem Polymer und Anschlußleitungen im Inneren des Polymers bzw. der Polymerschichten, aus z.B. Polyimid oder Silikon unter leichter Zugspannung, auf dem sich beidseitig Mikroelektroden aus Metall, z.B. Platin, Iridium, befinden. Die Mikroelektroden können aufgedampft oder gedruckt sein. Entlang der Längsachse der Nervenplatte sind 20-30 Reihen von Elektroden aufgebracht, die beliebig konfiguriert werden können, um die gleiche FNP sowohl prinzipiell für die Ableitung von Elektroneurogrammen als auch für die Stimulation einsetzen zu können. Von den Mikroelektroden führen Leiterbahnen im Inneren der Nervenplatte zu einem in dieselbe integrierten flexiblen, mehrlagigen Leiterband (Kabel), auf dem sich z.B. signalvorverarbeitende Mikrochips befinden können, ebenso Verstärker oder auch Antennen für eine induktive Ankopplung. Da bei Polyimid die genannten Bauteile nicht monolithisch integriert werden können, muß eine hybride Bauweise z.B. Bauelemente nach dem Stand der Technik zu Schaltungen aufgebaut, vorgesehen werden. Diese Bauteile mit der entsprechenden miniaturisierten Schaltung können gemäß Anspruch 9 auf dem Leiterband selbst angeordnet sein. Durch die integrierte Bauweise des Leiterbandes mit der Nervenplatte kann eine erhebliche Fehlerquelle, ein Kabelbruch an der Verbindungsstelle, eliminiert werden.

Das Leiterband kann andererseits auch durch die Haut geführt werden und die Ansteuer- und Signalempfangseinheit außerhalb am Körper getragen werden. Falls diese Bauteile auf dem Leiterband mitimplantiert sind, können durch induktive Ankopplung sowohl Energie als auch Signale in beide Richtungen (in den Körper und heraus) übertragen werden. Es ist auch möglich, einen Energieträger z.B. langlebige Batterien mitzuimplantieren. Auch dann erfolgt die Übertragung der Signale an eine außerhalb des Körpers angeordneten Elektronik insbesondere zur Auswertung der Elektroneurogramme.

Falls im wesentlichen stimuliert werden soll, und nur eine beschränkte Bearbeitung der empfangenen Elektroneurogramme nötig ist, z.B. bei der Elektrotherapie, kann die elektronische Verarbeitung auch in der auf dem Leiterband vorgesehenen Signalempfangseinheit direkt vorgenommen werden.

Weiterhin wird erfindungsgemäß nach Anspruch 10 das Implantat mittels Multilayertechnik hergestellt.

Für die Zeit des Prozesses wird ein Siliziumwafer oder ein anderer Träger quasi als "Prozessierungshilfe" benutzt, auf dem alle Prozeßschritte ablaufen. Abwechselnd wird eine dünne (2-20 µm) Polyimidschicht aus einem flüssigen Vorprodukt aufgeschleudert und durch Ausheizen ausgehärtet und eine Metallschicht in Dünnfilmtechnik aufgebracht und strukturiert. Den Abschluß bildet eine weitere Polyimidschicht. Das Polyimid des entstandenen Multilayeraufbaus wird ausschließlich mit Trockenätzverfahren (reaktives lonenätzen: RIE) strukturiert. Bei der Herstellung von vielen Nervenplatten auf einem Träger erfolgt die Vereinzelung der Nervenplatten ebenfalls mittels RIE, wodurch beliebige Formen bezüglich der äußeren Form in hoher Genauigkeit möglich werden, so auch das lange, integrierte Kabel an jeder Nervenplatte. Am Ende des Prozesses der Multilayer-Nervenplatte werden die Nervenplatten mit dem jeweils integrierten Kabel vom Siliziumwafer abgelöst. Ihre Dicke beträgt dann ca. 10-30 µm.

### Ausführungsbeispiel

Entlang der Längsachse der Nervenplatte sind 5 - 30 Reihen mit jeweils 2 - 20 Elektroden aufgebracht, deren Form und Position beliebig konfiguriert werden kann. Der Abstand der Elektroden beträgt dabei für die Elektroden zur Stimulation vorzugsweise 150 - 250 µm und für die Elektroden zur Ableitung der Signale 80 - 1 20 µm. So ist die gleiche flexible Nervenplatte sowohl prinzipiell für die Ableitung von Elektroneurogrammen (ENG) als auch für die Stimulation einsetzbar. Die Maße der flexiblen Nervenplatte selbst betragen ca. 2 mm x 12 mm zuzüglich der etwa 10 - 50 mm für das Kabel. Die Abbildung zeigt schematisch oben ein dreidimensionales Bild der Nervenplatte und unten eine Aufsicht.

Das Kabel, bzw. flexible Leiterband, ist mit den Leiterbahnen im Inneren der Nervenplatte und damit mit den Elektroden verbunden. Bei der Herstellung werden beide Typen von Leiterbahnen gleichzeitig nach dem Stand der Technik auf der untersten Polymerschicht aufgebracht, z.B. aufgedampft. Ebenso werden die Elektroden und die im Inneren der Nervenplatte befindlichen Leiterbahnen in einem Arbeitsschritt aufgebracht , wobei die Elektrodenschichten dicker ausgebildet sind.

Die Elektroden können auch konzentrisch von einer weiteren Elektrode umgeben sein, wobei z. B. die innere Elektrode als Sendeelektrode und die äußere Elektrode als Empfangselektrode ausgebildet sein kann und jeweils an die entsprechende Signalverarbeitungseinheit angeschlossen ist. Die Elektroden und Leitungen werden danach von einer weiteren Poymerschicht überzogen, und dann die Fenster zu den Elektroden erzeugt.

Die flexible Nervenplatte wird zwischen die Faszikel des Nervenbündels eingelegt, geklebt oder genäht. Bei etwa 2 - 10 Faszikel pro Nervenbündel und mehreren verwendeten flexiblen Nervenplatten ergeben sich so wesentlich mehr Plätze an denen die ca. 50.000 Nervenfasern (ca. 5 - 7000 Fasern pro Faszikel) angeregt bzw. stimuliert oder abgegriffen werden können.

Bei einer weiteren Ausführungsform werden auf die untere Metallschicht für die Elektroden eine Zwischenschicht aus dem Polymerwerkstoff und darauf eine zweite Metallschicht aufgebracht, sodaß zu jeder Seite der Nervenplatte eine ganze "Ebene" von Leiterbahnen gehört und somit die Leiterbahnen zu den Elektroden besser und einfacher isoliert werden können. Die Leiterbahnen werden mittels eines bekannten Lift-off-Verfahrens strukturiert, also z.B. vereinzelt, an deren Ende sich die jeweilige Elektrode befindet. Nach Aufbringung der Polymerdeckschicht werden die Fenster zu den Elektroden mittels des Trockenätzverfahrens geöffnet, wobei dies auch für beide Seiten der Nervenplatte durchgeführt werden kann, ebenso wie für die dreischichtige Nervenplatte.

Die Fenster sollten etwas kleiner als die Elektrodenfläche ausgebildet sein, damit die Elektroden im Verbund der Nervenplatte besser verankert sind.

### Weitere Ausführungsbeispiele

Zu berücksichtigen ist beim Einsatz der Funktionellen Elektrostimulation, z.B. über periphere Elektroden bei querschnittgelähmten Patienten, daß durch die Querschnittlähmung, die mit einem Wegfall hemmender Einflüsse übergeordneter zentralnervöser Informationen verbunden ist, stets Spastik in unterschiedlicher Stärke auftritt. Bevor eine motorische Stimulation sinnvoll und möglich ist, muß die Spastik gelöst werden. Ferner bereitet die Spastik durch Kontraktionen und die daraus entstehenden Schmerzen eine zuzüglich der Lähmung weitere Minderung der Lebensqualität für die Patienten.

Eine Möglichkeit zur Minderung der Spastik ist die Elektrotherapie. Hier ist ein Einsatz der FNP möglich, da durch die Kombination von Elektroden die gleichen großflächigen Reizungen ganzer Muskelgruppen möglich sind. Durch die implantierten Elektroden könnte die Therapie wesentlich vereinfacht werden und eine größere Patientenakzeptanz und Verbreitung finden. Somit wäre ein Einsatz und die Verbreitung der FNP schon möglich, bevor Konzepte zur Kodierung und Dekodierung im Rahmen der "Motorischen Neurotechnologie" ausgereift sind.

### Ein weiteres bevorzugtes Ausführungsbeispiel:

Die FNP könnte auch als Auditory Brainstem Implant Einsatz finden. Sie bietet eine größere Anzahl von Elektroden und eine gute Anpassung an die Oberfläche des Nucleus cochlearis. Damit die FNP ortsfest an der gewünschten Stelle bleibt, kann das Substrat auch als Siebstruktur ausgelegt werden, so daß durch einwachsende Fibroblasten eine Fixierung erfolgt. Eine Stimulation mit höherer räumlicher Auflösung läßt eine verbesserte Abbildung der Umweltgeräusche erwarten, die in Richtung für eine tatsächliche Spracherkennung zeigen kann.

Als Implantat im visuellen Cortex zeigt die FNP einen Mittelweg zwischen epidularen, d.h. auf der harten Hirnhaut (Dura Mater) liegenden, und intracortikalen Lösungen von Elektrodenkonfigurationen auf. Sie kann in den Arachnoidalraum eingebracht werden und sich aufgrund ihrer Flexibilität an die Hirnwindungen des visuellen Cortex (V1, Area 17) im Occipitallappen anpassen. Denkbar ist das Einbringen von je einer FNP in den Sulcus calcarinus beider Hirnhälften, der von der Sehrinde ausgekleidet wird. Bei dieser Konfiguration sind die Reizelektroden nur durch die weiche Hirnhaut (Pia Mater) von den zu reizenden Nervengebieten getrennt, ohne das empfindliche Gewebe der Hirnrinde jedoch direkt zu durchdringen und zu schädigen. Es besteht eine funktionelle Gliederung der Sehrinde in vertikal zur Oberfläche durch die ganze Breite des Cortex verlaufende Säulen von ca. 300-500 µm Durchmesser. Sie gehören zu umschriebenen Bereichen in der Netzhaut und sind visuotopisch angeordnet. Durch oberflächliche Reizung der vertikalen Säulen, die umschriebenen Bezirken in der Netzhaut zugeordnet sind, wird eine Erzeugung von Phosphenen erwartet, die durch die mit der FNP möglichen Elektrodendichte und -kombination eine Wahrnehmung von differenzierten Formen erlaubt.

## Patentansprüche

1. Flexible und nicht leitende, künstliche, implantierbare Nervenplatte mit einem E-Modul von 3000 - 1000 N/mm² und einer Dicke < 50 µm zum Einlegen und Einfügen zwischen die Faszikel eines Nervenbündels mit mehreren Elektroden auf beiden Seiten der Nervenplatte, die über Leitungen im Inneren der Nervenplatte mit einem mit der Nervenplatte integrierten Kabel verbunden sind, und dieses Kabel an eine Ansteuer- und Signalempfangseinheit anschließbar ist.

2. Nervenplatte nach Anspruch 1,
**dadurch gekennzeichnet,**
**daß** die Nervenplatte aus einem Polymerwerkstoff, vorzugsweise aus Polyimid oder Silicon besteht.

3. Nervenplatte nach Anspruch 1,
**dadurch gekennzeichnet,**
**daß** das E-Modul vorzugsweise 2500 - 2000 N/mm² und/oder die Dicke vorzugsweise < 20µm und diese bevorzugt < 10µm beträgt.

4. Nervenplatte nach Anspruch 1,
**dadurch gekennzeichnet,**
**daß** die Elektroden aus Platin, Iridium, Iridiumoxyd oder Gold bestehen und entweder an die Ansteuereinheit zur Stimulation der Nerven oder an die Signalempfangseinheit zum Empfang von Elektroneurogrammen, d.h. Nervensignale, angeschlossen sind.

5. Nervenplatte nach Anspruch 4,
**dadurch gekennzeichnet,**
**daß** die Sende- und Empfangselektroden getrennt auf beiden Seiten oder gemischt auf beiden Seiten angeordnet sind.

6. Nervenplatte nach Anspruch 4,
**dadurch gekennzeichnet,**
**daß** die Ansteuer- und Signalempfangseinheit derart ausgebildet ist, daß die Elektroden intermittierend mit Signalen zur Stimulation der Nerven beaufschlagbar und danach Elektroneurogramme empfangbar sind.

7. Nervenplatte nach Anspruch 1,
**dadurch gekennzeichnet,**
**daß** vorzugsweise 20 - 300 Elektroden pro Seite vorgesehen sind, die in Reihen und Spalten angeordnet und unabhängig in beliebiger Kombination mit Signalen beaufschlagbar sind oder auf Empfang geschaltet sind.

8. Nervenplatte nach Anspruch 1,
**dadurch gekennzeichnet,**
**daß** das Kabel als Flachbandkabel ausgebildet ist, und in mehreren Lagen mit der Nervenplatte integriert ist.

9. Nervenplatte nach Anspruch 4,
**dadurch gekennzeichnet,**
**daß** die Ansteuer- und Signalempfangseinheit Vorverstärker und/oder Verstärker und/oder Einrichtungen für eine induktive Ankopplung für Sender bzw. Empfänger außerhalb des Körpers aufweist, die in hybrider Bauweise auf oder an dem Flachbandkabel vorgesehen sind.

10. Verfahren zur Herstellung einer flexiblen, künstlichen Nervenplatte zum Einlegen oder Einfügen zwischen die Faszikel eines Nervenbündels mit mehreren Elektroden auf einer oder beiden Seiten der Nervenplatte mit Leitungen im Innern der Nervenplatte, und einem integrierten Kabel nach Anspruch 1,
**dadurch gekennzeichnet,**
**daß** die Nervenplatte in Multilayertechnik hergestellt ist.

## Claims

1. Flexible and non-conducting artificial implantable neural terminal plate with a modulus of elasticity of 3,000 to 1,000 N/mm² and a thickness of < 50µm for being placed and inserted between the fasciculi of a fascicle, including a plurality of electrodes on both sides of the neural terminal plate, which are connected via lines inside the neural terminal plate to a cable integrated with the neural terminal plate, wherein said cable is adapted to be connected to a controller and signal receiver unit.

2. Neural terminal plate according to Claim 1,
**characterised in**
**that** the neural terminal plate consists of a polymer material, preferably of polyimide or silicone.

3. Neural terminal plate according to Claim 1,
**characterised in**
**that** the modulus of elasticity amounts preferably to 2,500 to 2,000 N/mm² and/or that the thickness is preferably < 20 µm and the latter preferably < 10 µm.

4. Neural terminal plate according to Claim 1,
**characterised in**
**that** said electrodes consist of platinum, iridium, iridium oxide or gold and are connected either to said controller unit for stimulating the nerves or to said signal receiver unit for receiving electro-neurogram, i.e. nerve signals.

5. Neural terminal plate according to Claim 4,
**characterised in**
**that** said transmitter and receiver electrodes are separately disposed on both sides or in mixed form on both sides.

6. Neural terminal plate according to Claim 4,
**characterised in**
**that** said controller and signal receiver unit is so configured that the electrodes are adapted to be exposed intermittently to signals for stimulating the nerves and that subsequently electro-neurogram can be received.

7. Neural terminal plate according to Claim 1,
**characterised in**
**that** preferably 20 to 300 electrodes are provided per side, which are disposed in lines and columns whilst they are adapted to be exposed independently to signals in an optional combination or to be switched to receiving operation.

8. Neural terminal plate according to Claim 1,
**characterised in**
**that** said cable is configured as flat cable and is integrated in several layers with the neural terminal plate.

9. Neural terminal plate according to Claim 4,
**characterised in**
**that** said controller and signal receiver unit comprises pre-amplifiers and/or amplifiers and/or means for inductive coupling for transmitters or receivers, respectively, outside the body, which are provided in a hybrid configuration on or at said flat cable.

10. Method of manufacturing a flexible artificial neural terminal plate for placing or inserting it between the fasciculi of a fascicle, including a plurality of electrodes on one or both sides of the neural terminal plate with lines inside the neural terminal plate, and an integrated cable according to Claim 1,
**characterised in**
**that** the neural terminal plate is manufactured in a multi-layer technique.

## Revendications

1. Plaque de prise aux nerfs flexible et non-conductrice artificielle à implanter, ayant un module d'élasticité de 3.000 à 1.000 N/mm² et une épaisseur de < 50µm, apte à être placée et insérée entre les fascicules nerveux d'un faisceau nerveux, comprenant une pluralité d'électrodes des deux côtés de la plaque de prise aux nerfs, qui sont raccordées, via des lignes à l'intérieur de la plaque de prise aux nerfs, à un câble intégré avec la plaque de prise aux nerfs, dans laquelle ledit câble est apte à être raccordé à une unité de commande et de réception des signaux.

2. Plaque de prise aux nerfs selon la revendication 1,
**caractérisée en ce**
**que** la plaque de prise aux nerfs consiste en un matériau en polymère, de préférence en polyimide ou silicone.

3. Plaque de prise aux nerfs selon la revendication 1,
**caractérisée en ce**
**que** le module d'élasticité correspond, de préférence, à 2.500 à 2.000 N/mm² et/ou en ce que l'épaisseur correspond, de préférence, à une valeur < 20 µm, en étant préférablement < 10 µm.

4. Plaque de prise aux nerfs selon la revendication 1,
**caractérisée en ce**
**que** lesdites électrodes consistent en platine, iridium, l'oxyde de l'iridium ou en or, en étant raccordées soit à ladite unité de commande afin de stimuler les nerfs, soit à ladite unité de réception des signaux afin de recevoir l'électro-neurogramme, c'est-à-dire les signaux nerveux.

5. Plaque de prise aux nerfs selon la revendication 4,
**caractérisée en ce**
**que** lesdites électrodes d'émission et de réception sont disposées séparément des deux côtés ou en forme mixte des deux côtés.

6. Plaque de prise aux nerfs selon la revendication 4,
**caractérisée en ce**
**que** ladite unité de commande et de réception des signaux est configurée de façon, que les électrodes sont aptes à être exposé, de façon intermittente, aux signaux afin de stimuler les nerfs, et en ce qu'ensuite on peut recevoir l'électro-neurogramme.

7. Plaque de prise aux nerfs selon la revendication 1,
**caractérisée en ce**
**que**, de préférence, 20 à 300 électrodes sont disposées par côté, qui sont arrangées en lignes et colonnes, pendant qu'elles sont aptes à être exposé indépendamment aux signaux en une combinaison à volonté ou à être commuté en régime de réception.

8. Plaque de prise aux nerfs selon la revendication 1,
**caractérisée en ce**
**que** ledit câble est configuré sous forme d'un câble plat, en étant intégré en plusieurs couches avec la plaque de prise aux nerfs.

9. Plaque de prise aux nerfs selon la revendication 4,
**caractérisée en ce**
**que** ladite unité de commande et de réception des signaux comprend des préamplificateurs et/ou des amplificateurs et/ou des moyens de couplage inductif pour des émetteurs ou respectivement récepteurs à l'extérieur du corps, qui sont disposés dans une configuration hybride sur ledit ou audit câble plat.

10. Procédé de fabrication d'une plaque de prise aux nerfs flexible artificielle à être placé ou inséré entre les fascicules nerveux d'un faisceau nerveux, comprenant une pluralité d'électrodes d'un côté ou des deux côtés de la plaque de prise aux nerfs, aux lignes à l'intérieur de la plaque de prise aux nerfs, ainsi qu'un câble intégré, selon la revendication 1,
**caractérisée en ce**
**que** la plaque de prise aux nerfs est fabriquée moyennant une technique multicouche.
